# EUROPEAN PATENT SPECIFICATION

(11) **EP 1 274 355 B1**
(45) Date of publication and mention of the grant of the patent: **14.07.2004**
(21) Application number: 01929582.3
(22) Date of filing: 19.04.2001
(51) Int. Cl.: A61B 17/66

(54) **APPARATUS FOR INTRAORAL DISTRACTION OSTEOTOMY TO WIDEN THE LOWER JAW**
GERÄT ZUR INTRAORALEN DISTRAKTIONSOSTEOSYTHESE ZUM ERWEITERN DES UNTERKIEFERS
APPAREIL D'OSTEOSYNTHESE PAR DISTRACTION INTRA-BUCCALE PERMETTANT D'ELARGIR LA MACHOIRE INFERIEURE

(30) Priority: 19.04.2000 BE 200000282
(43) Date of publication of application: 15.01.2003
(73) Proprietor: Mommaerts, Maurice Yves, 9830 Sint-Martens-Latem (BE)
(72) Inventor: Mommaerts, Maurice Yves, 9830 Sint-Martens-Latem (BE)
(74) Representative: De Hoop, Eric
(86) International application number: PCT/EP2001/004440
(87) International publication number: WO 2001/080752

(56) References cited:
- EP-A- 1 099 415
- WO-A-99/04715
- DE-A- 2 440 856
- DE-A- 19 731 921
- US-A- 5 902 304
- US-A- 6 007 535

## Description

The present invention pertains to a distraction device used to broaden the lower jaw (mandible) after osteotomy and fixation of an adjustable bridging onto the bone of the mandibular symphysis with the goal to increase the distance between the two mutually opposite anterior halves of the jaw by means of gradual expansion.
The device is used in maxillofacial surgery, in particular in orthognathic surgery in patients with a narrow anterior mandible. It concerns a developmental disturbance, with too little space in the arch for the teeth present, with potentially a cross bite, with potentially too much space between the cheek and the dental arches, with ant upper jaw that can be too narrow as well, with potentially a malocclusion with the upper dental arch, with potentially too little space for the tongue in the oral cavity, and with potentially a psychological aesthetic problem. The aim of the intervention is to avoid tooth extractions to provide space for the teeth on the jaw, to restore the occlusion of the teeth, to induce a favourable tongue position, to improve smile aesthetics and to bring the face into proportions.
The assets of the invention compared to the apparatus of GUERRERO en BELL (WO 98/25538) are that the dissection and fixation are limited to the osteotomy region, that in the middle part of the device a weakening is provided that allows a stretching along an arch segment without loosing rigidity, and that activation on two different levels allows controlled differential movements of the jaw segments. It has the advantage over tooth-anchored devices, that present apparatus does not have to be custom made and that the tongue is not hindered at the inner side by the lingually placed activation part, and that bone fixation does not entail the risk of loosening.

This is realised according to the invention because the device has following parts.
two vertical fixation arms, that are fixed to the mandibular bone left and right from an osteotomy-line and fixed under the mucous membranes, and which have above the mucous membranes two horizontal bores with an opposing screw thread;
two horizontal distraction cylinders hat are provided at their end with an opposite running screw thread and in the middle with a pliable weakening. The cylinders fit in the horizontal bores at the top of the vertical fixation arms.

The distraction part consists of two horizontal supraposed distraction cylinders with a central weakening, in one design in the form of a spiral, enabling curved distraction along an arch segment. The stretching allows a rotation of the jaw halves and prohibits a pure translation that is potentially harmful for the temporo-mandibular joint function. In the plane of the two cylinders, the stiffness is sufficient to withstand the vertical forces that play on the jaw.
After a latency period of four to seven days, the activation can start. The activation comprises 1/2 mm daily. This is realised with a key that fits into a hole of the side of the cylinder. After the distraction period that lasts few weeks, and the stabilisation period of one to three months, the orthodontist can start with fixed orthodontic appliances. One to three months later, the distractor can be removed. Complete bony consolidation happens in the distraction area.
Advantageously, the distraction cylinder is a rod, which has at both sides of the central weakening an opposite running screw thread.

The device is made of titanium. This material is well known for its optimum biocompatibilty, both for incorporation into the bone (osteointegration) and for incorporation into the soft tissue parts. No allergies to titanium have been described. Dimetalosis has been ruled out because the complete device and the fixation screws consist of titanium.

These and other characteristics of the invention will emerge from the following description, in which reference is made to the appended drawings, which show an exemplary embodiment of an apparatus according to the invention, and in which:
Figure 1 shows a front view, looking from outside the mouth inwards of a distraction apparatus in the extended position on the chin according to the invention on a scale of 1:1;
Figure 2 shows a frontal view of an apparatus in a contracted position on a scale of 2:1;
Figure 3: a side view of a fixation arm on a scale of 2:1;
Figure 4: a front view of a distraction cylinder on a scale of 4:1;

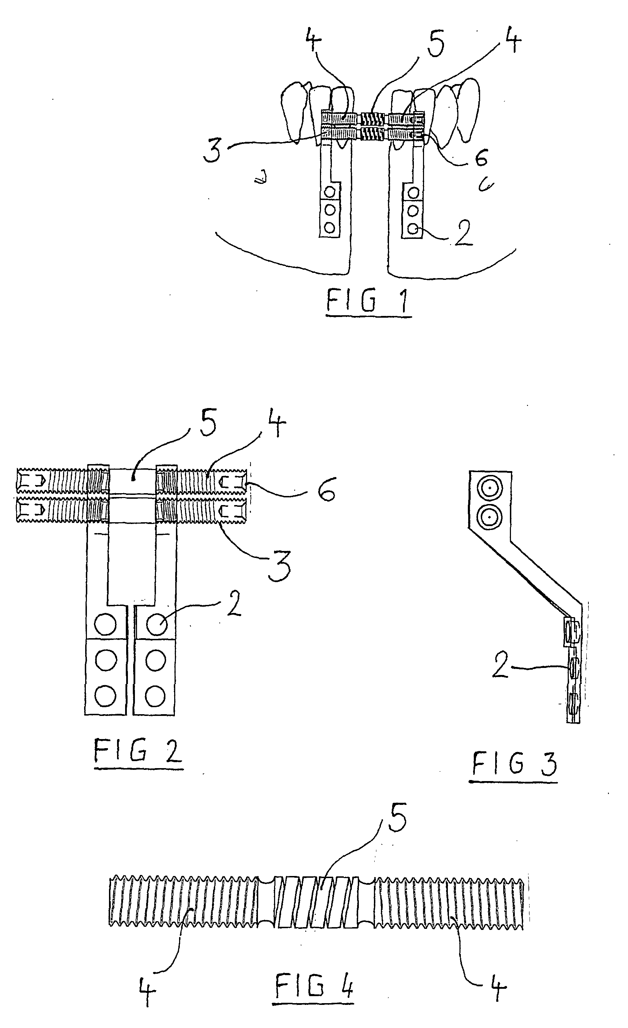

In these figures, identical numerals refer to identical or similar elements.
A distraction osteotomy is a surgical operation for correcting deformity or curvature of a bone which is curved or too short, the bone being split into at least two bone segments being joined together again by dynamic osteosynthesis, that is to say gradual repositioning and fixation of the bone segments with the aid of guided rods, small metal plates, screws, pins, bone pegs or hoops.
The distration apparatus depicted in Figure 1 and 2, serves to broaden the lower jaw, according to an application in the chin area, after a vertical osteotomy, which is partly or totally positioned between the fixation arms. It comprises the following parts:
- two fixation arms 1, fixed with screws 2 in bores at the outer side of the lower jaw, left and right of a vertical osteotomy;
a distraction cylinder 3, depicted in Figure 4, in different lengths.
A distraction cylinder is a rod that has at both extremities over a certain length a screw thread 4 of opposite direction. The middle part of the cylinder is limited bendable. The distraction cylinders 3 are placed parallel one above the other. On one of both sided the distraction cylinders 3 is a polygonal hole 6, fitting an activation key.

## Claims

1. Distraction apparatus for the broadening of the anterior lower jaw after osteotomy with application of the forces directly to the bone of the lower jaw with the aim to reconstruct the jaw by increasing the distances between the two opposing bone segments by means of gradual stretching **characterised in that** it consists of following parts: two vertical fixation arms (1), that are to be fixed to the lower jaw by means of screws (2), and of which the part piercing the soft tissues is provided of two horizontal openings in which two distraction cylinders (3) are screwed; said distraction cylinders (3) being expansion screws, with an opposing screw thread at each side;

2. Distraction apparatus according to one of the proceeding claim 1, **characterised in that** the expansion screws (3) are mechanical jackscrews, with a pliable mid-part (5), allowing expansion along an arch segment with minimally counteractive force vectors on the fixation parts.

3. Distraction apparatus according to claims 1 and 2, **characterised in that** the expansion screws (3) , are placed parallel one above the other, by which forces acting in the plane of both cylinders will provoke less distortion per cylinder, c.q. the whole apparatus and the jaw segments.

4. Distraction apparatus according to one of the proceeding claims, **characterised in that** the expansion screws (3), are placed parallel one above the other and can be turned separately, enabling differential activation and expansion of the bone segments in a plane parallel to that of the two distraction cylinders.

## Patentansprüche

1. Distraktionsvorrichtung für die Verbreiterung des Vorderunterkiefers nach Osteotomie mit Angriff der Kräfte direkt auf dem Unterkieferknocken im Hinblick auf eine Rekonstruktion des Kiefers durch Zunahme der Abstände zwischen den zwei gegenüberliegenden Knochensegmenten durch allmähliche Streckung, **dadurch gekennzeichnet, daß** es aus den nachfolgenden Teilen besteht:
zwei vertikale Fixierungsärme (1), die durch Schrauben (2) an dem Unterkiefer befestigt werden müssen, und wovon das durch die weichen Geweben steckende Teil mit zwei horizontalen Öffnungen, worin die Distraktionszylinder (3) geschraubt werden, versehen ist;
Distraktionszylinder (3), welche Expansionsschrauben sind, mit einem gegenüberliegenden Gewinde an jeder Seite.

2. Distraktionsvorrichtung nach Anspruch 1, **dadurch gekennzeichnet, daß** die Expansionsschrauben (3) mechanische Schraubwinden sind, mit einem geschmeidigen Mittelstück (5), wodurch Expansion entlang einem Bogensegment mit minimal entgegenwirkenden Kraftvektoren auf den Fixierungsteilen geschehen kann.

3. Distraktionsvorrichtung nach Ansprüchen 1 und 2, **dadurch gekennzeichnet, daß** die Expansionsschrauben (3) parallel obeneinander angeordnet werden, wodurch auf die Fläche der beiden Zylinder einwirkende Kräfte weniger Biegung pro Zylinder, ggf der ganzen Vorrichung und der Kiefersegmente, verursachen werden.

4. Distraktionsvorrichtung nach einem der vorhergehenden Ansprüche, **dadurch gekennzeichnet, daß** die Expansionsschrauben (3) parallel obeneinander angeordnet sind und getrennt gedreht werden können, wodurch differentielle Aktivierung und Expansion der Knochensegmente in der Fläche parallel zu der der zwei Distraktionszylinder erreicht werden.

## Revendications

1. Distracteur destiné à élargir la mâchoire inférieure antérieure après ostéotomie, en appliquant les forces directement sur l'os de la mâchoire inférieure, dans le but de reconstruire la mâchoire en augmentant les distances entre les deux segments osseux opposés, par étirement progressif, **caractérisé en ce qu'**il est constitué des composants suivants : deux bras de fixation verticaux (1), destinés à être fixés sur la mâchoire inférieure au moyen de vis (2), et dont la partie qui perce les tissus mous présente deux ouvertures horizontales dans lesquelles sont vissés deux cylindres distracteurs (3) ;
ces cylindres distracteurs (3) étant des vis à expansion et présentant un filetage opposé de chaque côté.

2. Distracteur selon la revendication 1, **caractérisé en ce que** les vis à expansion (3) sont des vis de calage mécaniques, présentant une partie centrale flexible (5) permettant l'expansion le long d'un segment arqué en exerçant des vecteurs de force d'un antagonisme minimal sur les parties de fixation.

3. Distracteur selon les revendications 1 et 2, **caractérisé en ce que** les vis à expansion (3) sont montées parallèles, l'une au-dessus de l'autre, ce qui permet que les forces qui agissent dans le plan des deux cylindres provoquent une distortion moins importante, par cylindre, casu quo l'ensemble de l'appareil et les segments de mâchoire.

4. Distracteur selon l'une des revendications précédentes, **caractérisé en ce que** les vis à expansion (3) sont placées de façon parallèle l'une au-dessus de l'autre, et peuvent être tournées séparément, permettant une activation et une expansion différentes des segments osseux dans un plan parallèle à celui des deux cylindres distracteurs.
